(19) Europäisches Patentamt
European Patent Office
Office européen des brevets

(11) Publication number: 0 161 811
B1

(12) EUROPEAN PATENT SPECIFICATION

(45) Date of publication of patent specification: 28.11.90

(51) Int. Cl.[5]: A 61 K 7/08, C 11 D 3/33, C 11 D 1/835, C 11 D 1/75

(21) Application number: 85302614.4

(22) Date of filing: 15.04.85

# (54) Composition for use as a hair-rinse.

(30) Priority: 16.04.84 JP 76199/84

(43) Date of publication of application:
21.11.85 Bulletin 85/47

(45) Publication of the grant of the patent:
28.11.90 Bulletin 90/48

(84) Designated Contracting States:
DE FR GB

(56) References cited:
GB-A-1 159 226
US-A-3 697 452
US-A-4 259 217
US-A-4 264 479

H. JANISTYN: "Handbuch der Kosmetika und Riechstoffe", vol. III: "Die Körperpflegemittel", 2nd edition, 1973, pages 278-279, Dr. Alfred Hüthig Verlag, Heidelberg, DE; "Haar-Shampoos mit konditionierenden Eigenschaften"

(73) Proprietor: NIPPON OIL AND FATS COMPANY, LIMITED
10-1, Yuraku-cho 1-chome
Chiyoda-ku Tokyo (JP)

(72) Inventor: Akimoto, Shin-Ichi
No. 1326-28 Zushimachi
Machida-shi Tokyo (JP)
Inventor: Suginaka, Akinori
No. 1598-12 Hagizono
Chigasaki-shi Kanagawa (JP)

(74) Representative: Perry, Robert Edward et al
GILL JENNINGS & EVERY 53-64 Chancery Lane
London WC2A 1HN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 161 811 B1

**Description**

The present invention relates to a composition which can be used as a hair-rinse.

Hair-rinses should soften hair, in order to allow smooth combing, prevent electrostatic charges, render the hair supple and lustrous, and protect the hair. Cationic surface-active agents are known to satisfy these criteria and are therefore often used, together with components such as moisturising agents, oils or fats. Hair-rinses containing cationic surface-active agents, however, are generally marketed in a form having a transparent or turbid appearance. Moreover, hair-rinse compositions containing a cationic surface-active agent often irritate the skin.

It would be desirable to provide hair-rinse compositions which are lustrous. Compounds such as ethylene glycol distearate have been used for this purpose, but their effect is poor. It is also difficult to control the manufacture of compositions containing such compounds.

A hair-rinse composition according to the present invention having a pearly lustre, consists of:

(1) 1 part by weight of a $C_{16-45}$ cationic surface-active agent of the formula

$$R^1R^2R^3R^4N^{\oplus}\ X^{\ominus}$$

wherein $R^1$, $R^2$, $R^3$ and $R^4$ are independently selected from $C_{1-24}$ hydrocarbyl and $C_{1-24}$ hydroxyalkyl, and X is a halogen atom;

(2) from 0.1 to 10 parts by weight of a $C_{16-45}$ amine oxide of the formula

$$R^5R^6R^7N \rightarrow O$$

wherein $R^5$, $R^6$ and $R^7$ are independently selected from $C_{1-24}$ hydrocarbyl and $C_{1-24}$ hydroxyalkyl;

(3) from 0.01 to 2 parts by weight of an amino-acid having an isoelectric point not higher than 7; and

(4) from 0.01 to 2 parts by weight of an alkali metal or alkaline earth metal salt.

Examples of $C_{1-24}$ hydrocarbyl are methyl, ethyl, propyl, isopropyl, allyl, butyl, isobutyl, tert-butyl, amyl, isoamyl, hexyl, heptyl, octyl, 2-ethylhexyl, nonyl, decyl, undecyl, lauryl, tridecyl, myristyl, cetyl, isocetyl, stearyl, isostearyl, oleyl, octyldodecyl, behenyl, docosenyl, decyltetradecyl and benzyl. Examples of $C_{1-24}$ hydroxyalkyl are hydroxyethyl, hydroxypropyl, hydroxybutyl and 2,3-dihydroxypropyl. X, the halogen atom, is preferably chlorine or bromine.

The total number of carbon atoms in each of the surface-active agent and the amine oxide should not be less than 16, since the rinsing action will be lost owing to high water-solubility, nor more than 45, since then the water-dispersibility is low. $R^5$, $R^6$ and $R^7$ may be the same as $R^1$, $R^2$ and $R^3$, respectively.

Examples of suitable cationic surface-active agents are trimethylcetylammonium chloride, trimethylstearylammonium chloride, trimethylbehenylammonium chloride, trimethyldocosenylammonium chloride, dimethyldicetylammonium chloride, dimethyldistearylammonium chloride, trimethyloctyldodecylammonium chloride and trimethyldecyltetradecylammonium chloride.

Preferred amine oxides are N,N-dimethylcetylamine oxide, N,N-dimethylstearylamine oxide, N,N-di(2-hydroxyethyl)stearylamine oxide, N-methyldistearylamine oxide, N,N-dimethylbehenylamine oxide, N,N-dimethyldocosenylamine oxide, N,N-dimethyloctyldodecylamine oxide and N,N-dimethyldecyltetradecyl-amine oxide.

The amino-acid is, for example, glycine, alanine, valine, leucine, isoleucine, serine, threonine, phenylalanine, tyrosine, tryptophan, cysteine, cystine, methionine, proline, hydroxyproline, asparaginic acid, glutamic acid, asparagine or glutamine. Its isoelectric point should not exceed 7, since otherwise it may not dissolve in the composition, and give a solid deposit.

The alkali metal or alkaline earth metal salt is, for example, a salt of a strong acid such as hydrochloric acid, sulphuric acid or phosphoric acid with sodium, potassium, magnesium or calcium, or an alum.

Compositions of the invention may comprise, if desired, one or more of any of the four defined components. They may also contain other components of the type employed in conventional hair-rinse liquids, such as, for example, flavours, colouring agents, nutrients, oily substances, thickeners and refrigerants. Especially for use as a hair-rinse liquid, compositions of the invention may be marketed in a form dispersed in a solvent such as water or ethanol. The content of the surface-active agent may then be from 0.5 to 10, preferably from 1 to 7, % by weight.

Compositions of the invention have a pearly lustre capable of being maintained on storage under warm or cold conditions. They can be applied as such to the hair by hand, without irritating skin or hair. They can soften hair, give smooth combing, prevent electrostatic charge, impart glossy and supple appearance to hair, and protect hair.

The following Examples illustrate the invention. All percentages are by weight.

Example 1

Compositions were formulated from the components given in Table 1, the remainder being deionised water. Each formulation was heated to 70 C, to form a homogeneous liquid. This liquid was divided into two parts; the appearance of each was observed after they had been cooled to 20 C. These two samples

were then stored, each in a constant temperature bath of 40 C and 0 C, respectively, for 90 days, and their changes in appearance were observed.

The results are summarised in Table 1. Only compositions in accordance with the invention had a stable pearly appearance.

TABLE 1

| Test No. | Component | | | | | | | | | Pearly luster***) | | |
|---|---|---|---|---|---|---|---|---|---|---|---|---|
| | A (Cation. surfactant) | (%) | B (Amine oxide) | (%) | C (Amino acid) | (%) | D (Salt) | (%) | Other (%) | Direct. after | At 40°C | At 0°C |
| 1*) | 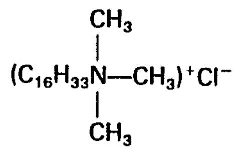 | (2.0) | 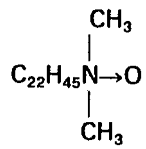 | (2.0) | Asparaginic acid | (0.4) | NaCl | (0.2) | — | ○ | ○ | ○ |
| 2*) | 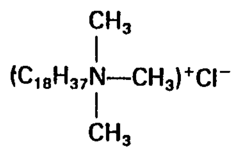 | (2.2) | 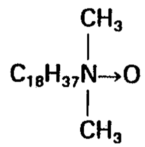 | (1.8) | Glutamic acid | (0.3) | $MgSO_4$ | (0.1) | — | ○ | ○ | ○ |
| 3*) | 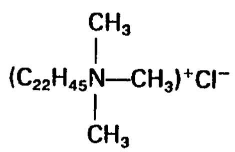 | (1.8) | 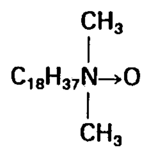 | (3.0) | Glutamic acid<br>Glycine | (0.4)<br>(0.1) | KCl | (0.2) | — | ○ | ○ | ○ |
| 4*) | 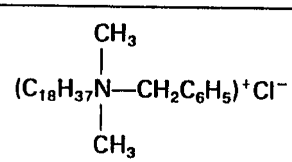 | (1.5) | 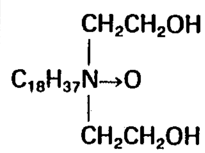 | (2.8) | Asparaginic acid | (0.2) | $Na_2SO_4$ | (0.4) | — | ○ | ○ | ○ |
| 5*) | 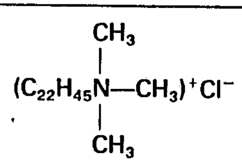 | (1.6) | 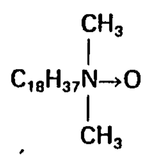<br>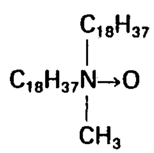 | (1.0)<br>(0.5) | Asparaginic acid | (0.2) | $Na_2SO_4$ | (0.3) | Ethanol (8.0) | ○ | ○ | ○ |

TABLE 1 (Cont'd)

| Test No. | Component | | | | | Pearly luster***) | | |
|---|---|---|---|---|---|---|---|---|
| | A (Cation. surfactant) (%) | B (Amine oxide) (%) | C (Amino acid) (%) | D (Salt) (%) | Other (%) | Direct. after | At 40°C | At 0°C |
| 6*) | $CH_3$<br>\|<br>$(C_{22}H_{43}N—CH_3)^+Cl^-$ (3.0)<br>\|<br>$CH_3$ | $CH_3$<br>\|<br>$C_{18}H_{37}N→O$ (1.0)<br>\|<br>$CH_3$<br>$CH_3$<br>\|<br>$C_{18}H_{33}N→O$ (0.7)<br>\|<br>$CH_3$ | Glutamic acid (0.1) | $K_2HPO_4$ (0.1)<br>KCl (0.1) | — | ○ | ○ | ○ |
| 7**) | Same to that of No. 2 | Same to that of No. 2 | Same to No. 2 | — | — | Δ | X | Δ |
| 8**) | Same to that of No. 2 | Same of that of No. 2 | — | Same to No. 2 | — | Δ | X | Δ |
| 9**) | Same to that of No. 2 | — | Same to No. 2 | Same to No. 2 | — | X | X | X |
| 10**) | — | Same to that of No. 2 | Same to No. 2 | Same to No. 2 | — | Δ | X | Δ |
| 11**) | Same to that of No. 3 | Same to that of No. 3 | Same to No. 3 | — | Ethylene-glycol distearate (1.0) | Δ | Δ | X |
| 12**) | Same to that of No. 3 | Same to that of No. 3 | — | Same to No. 3 | Same to the above | Δ | Δ | X |
| 13**) | Same to that of No. 3 | — | Same to No. 3 | Same to No. 3 | Same to the above | X | Δ | X |
| 14**) | — | Same to that of No. 3 | Same to No. 3 | Same to No. 3 | Same to the above | Δ | Δ | X |

*): Samples according to the present invention
**): Samples of Comparison Products
***): Evaluation of pearly luster: ○: Luster of pearly appearance; Δ: Weak pearly luster; X: No pearly luster

Example 2

A hair-rinse liquid was formulated from

| | |
|---|---|
| $(C_{18}H_{37})_2(CH_3)_2NCl$ | 1.6% |
| $(C_{18}H_{37})(CH_2CH_2OH)_2NO$ | 2.5% |
| Asparaginic acid | 0.3% |
| NaCl | 0.4% |
| Colour, flavour and antiseptic | adequate amounts |
| Deionised water, q.s. | 100% |

The liquid exhibited a pearly lustre on and after storage for 90 days at room temperature. It was substantially non-irritative, and hair rinsed with this liquid was soft, supple and satisfactorily lustrous.

Example 3

A hair-rinse liquid was formulated from

| | |
|---|---|
| $(C_{22}H_{45})(CH_3)_3NCl$ | 2.8% |
| $(C_{18}H_{37})(CH_3)_2NO$ | 3.2% |
| Glutamic acid | 0.4% |
| $Na_2SO_4$ | 0.6% |
| Colour, flavour and antiseptic | adequate amounts |
| Deionised water, q.s. | 100% |

The liquid exhibited a pearly lustre on and after storage for 90 days at room temperature. It was substantially non-irritative, and hair rinsed with this liquid was soft, supple and satisfactorily lustrous.

Example 4

A hair-rinse liquid was formulated from

| | |
|---|---|
| $(C_{22}H_{45})(CH_3)_3NCl$ | 2.26% |
| $(C_{18}H_{37})(CH_3)_2NO$ | 2.74% |
| Cetyl alcohol | 0.88% |
| Glutamic acid | 0.27% |
| $Na_2SO_4$ | 0.50% |
| Colour, flavour and antiseptic | adequate amounts |
| Deionised water, q.s. | 100% |

The liquid exhibited a pearly lustre on and after storage for 150 days at 0 to 40°C. It was substantially non-irritative, and hair rinsed with this liquid was soft, supple and satisfactorily lustrous.

**Claims**

1. A hair-rinse composition having a pearly lustre, consisting of:

(1) 1 part by weight of a $C_{16-45}$ cationic surface-active agent of the formula

$$R^1R^2R^3R^4N^{\oplus}X^{\ominus}$$

wherein $R^1$, $R^2$, $R^3$ and $R^4$ are independently selected from $C_{1-24}$ hydrocarbyl and $C_{1-24}$ hydroxyalkyl, and X is a halogen atom;

(2) from 0.1 to 10 parts by weight of a $C_{16-45}$ amine oxide of the formula

$$R^5R^6R^7N\rightarrow O$$

wherein $R^5$, $R^6$ and $R^7$ are independently selected from $C_{1-24}$ hydrocarbyl and $C_{1-24}$ hydroxyalkyl;

(3) from 0.01 to 2 parts by weight of an amino-acid having an isoelectric point not higher than 7; and

(4) from 0.01 to 2 parts by weight of an alkali metal or alkaline earth metal salt.

2. A composition according to claim 1, wherein $R^1$, $R^2$, $R^3$, $R^4$, $R^5$, $R^6$ and $R^7$ are independently selected from methyl, ethyl, propyl, isopropyl, allyl, butyl, isobutyl, tert-butyl, amyl, isoamyl, hexyl, heptyl, octyl, 2-ethylhexyl, nonyl, decyl, undecyl, lauryl, tridecyl, myristyl, cetyl, isocetyl, stearyl, isostearyl, oleyl, octyldodecyl, behenyl, docosenyl, decyltetradecyl and benzyl, hydroxyethyl, hydroxypropyl, hydroxybutyl and 2,3-dihydroxypropyl.

3. A composition according to claim 1 or claim 2, wherein X is Cl or Br.

4. A composition according to claim 3, wherein the surface-active agent is selected from trimethylcetylammonium chloride, trimethylstearylammonium chloride, trimethylbehenylammonium chloride, trimethyldocosenylammonium chloride, dimethyldicetylammonium chloride, dimethyldistearylammonium chloride, trimethyloctyldodecylammonium chloride and trimethyldecyltetradecylammonium chloride.

5. A composition according to any preceding claim, wherein the amine oxide is selected from N,N-dimethylcetylamine oxide, N,N-dimethylstearylamine oxide, N,N-di(2-hydroxyethyl)stearylamine oxide, N-methyldistearylamine oxide, N,N-dimethylbehenylamine oxide, N,N-dimethyldocosenylamine oxide, N,N-dimethyloctyldodecylamine oxide and N,N-dimethyldecyltetradecylamine oxide.

6. A composition according to any preceding claim, wherein the amino-acid is selected from glycine, alanine, valine, leucine, isoleucine, serine, threonine, phenylalanine, tyrosine, tryptophan, cysteine, cystine, methionine, proline, hydroxyproline, asparaginic acid, glutamic acid, asparagine and glutamine.

7. A composition according to any preceding claim, wherein the salt is selected from alums and the salts of sodium, potassium, magnesium or calcium with hydrochloric sulphuric or phosphoric acid.

8. A method which comprises rinsing hair with a liquid composition having a pearly lustre, comprising components (1), (2), (3) and (4) as defined in any preceding claim.

**Patentansprüche**

1. Haarspülpräparat mit perlartigem Glanz, bestehend aus

(1) 1 Gew.-Teil eines kationischen oberflächenaktiven $C_{16-45}$-Mittels der Formel

$$R^1R^2R^3R^4N^+X^-$$

worin $R^1$, $R^2$, $R^3$ und $R^4$ unabhängig ausgewählt sind aus $C_{1-24}$-Hydrocarbyl und $C_{1-24}$-Hydroxyalkyl und X ein Halogenatom ist,

(2) 0,1 bis 10 Gew.-Teilen eines $C_{16-45}$-Aminoxids der Formel

$$R^5R^6R^7N{\rightarrow}O$$

worin $R^5$, $R^6$ und $R^7$ unabhängig ausgewählt sind aus $C_{1-24}$-Hydrocarbyl und $C_{1-24}$-Hydroxyalkyl,

(3) 0,01 bis 2 Gew.-Teilen einer Aminosäure mit einem isoelektrischen Punkt nicht über 7 und

(4) 0,01 bis 2 Gew.-Teilen eines Alkalimetall- oder Erdalkalimetallsalzes.

2. Präparat nach Anspruch 1, worin $R^1$, $R^2$, $R^3$, $R^4$, $R^5$, $R^6$ und $R^7$ unabhängig ausgewählt sind aus Methyl, Ethyl, Propyl, Isopropyl, Allyl, Butyl, Isobutyl, t-Butyl, Amyl, Isoamyl, Hexyl, Heptyl, Octyl, 2-Ethylhexyl, Nonyl, Decyl, Undecyl, Lauryl, Tridecyl, Myristyl, Cetyl, Isocetyl, Stearyl, Isostearyl, Oleyl, Octyldodecyl, Behenyl, Docosenyl, Decyltetradecyl und Benzyl, Hydroxyethyl, Hydroxypropyl, Hydroxybutyl und 2,3-Di-hydroxypropyl.

3. Präparat nach Anspruch 1 oder 2, worin X Cl oder Br ist.

4. Präparat nach Anspruch 3, worin das oberflächenaktive Mittel aus Trimethylcetylammoniumchlorid, Trimethylstearylammoniumchlorid, Trimethylbehenylammoniumchlorid, Trimethyldocosenylammoniumchlorid, Dimethyldicetylammoniumchlorid, Dimethyldistearylammoniumchlorid, Trimethyloctyldodecylammoniumchlorid und Trimethyldecyltetradecylammoniumchlorid ausgewählt ist.

5. Präparat nach irgendeinem vorhergehenden Anspruch, worin das Aminoxid aus N,N-Dimethylcetylaminoxid, N,N-Dimethylstearylaminoxid, N,N-Di(2-hydroxyethyl)stearylaminoxid, N-Methyldistearylaminoxid, N,N-Dimethylbehenylaminoxid, N,N-Dimethyldocosenylaminoxid, N,N-Dimethyloctyldodecylaminoxid und N,N-Dimethyldecyltetradecylaminoxid ausgewählt ist.

6. Präparat nach irgendeinem vorhergehenden Anspruch, worin die Aminosäure aus Glycin, Alanin, Valin, Leucin, Isoleucin, Serin, Threonin, Phenylalanin, Tyrosin, Tryptophan, Cystein, Cystin, Methionin, Prolin, Hydroxyprolin, Asparaginsäure, Glutaminsäure, Asparagin und Glutamin ausgewählt ist.

7. Präparat nach irgendeinem vorhergehenden Anspruch, worin das Salz aus Alaunen und den Salzen von Natrium, Kalium, Magnesium oder Calcium mit Salz-, Schwefel- oder Phosphorsäure ausgewählt ist.

8. Verfahren, bei welchem Haar mit einem flüssigen Präparat mit perlartigem Glanz, das die Komponenten (1), (2), (3) und (4) gemäß Definition in irgendeinem vorhergehenden Anspruch umfaßt, gespült wird.

**Revendications**

1. Une composition pour le rinçage des cheveux ayant un lustre nacré, consistant en

(1) 1 partie en poids d'un agent tensioactif cationique en $C_{16}$—$C_{45}$ de formule

$$R^1R^2R^3R^4N^+X^-$$

dans laquelle $R^1$, $R^2$, $R^3$ et $R^4$ sont choisis indépendamment parmi un groupe hydrocarbyle en $C_1$—$C_{24}$ et un groupe hydroxyalkyle en $C_1$—$C_{24}$ et X est un atome d'halogène;

(2) de 0,1 à 10 parties en poids d'un oxyde d'amine en $C_{16}$—$C_{45}$ de formule

$$R^5R^6R^7N \rightarrow O$$

dans laquelle $R^5$, $R^6$ et $R^7$ sont choisis indépendamment parmi un groupe hydrocarbyle en $C_1$—$C_{24}$ et un groupe hydroxyalkyle en $C_1$—$C_{24}$.

(3) de 0,01 à 2 parties en poids d'un aminoacide ayant un point isoélectrique au plus égal à 7; et

(4) de 0,01 à 2 parties en poids d'un sel de métal alcalin ou alcalino-terreux.

2. Une composition selon la revendication 1, dans laquelle $R^1$, $R^2$, $R^3$, $R^4$, $R^5$, $R^6$ et $R^7$ sont choisis indépendamment parmi les groupes méthyle, éthyle, propyle, isopropyle, allyle, butyle, isobutyle, tert-butyle, amyle, isoamyle, hexyle, heptyle, octyle, 2-éthylhexyle, nonyle, décyle, undécyle, lauryle, tridécyle, myristyle, cétyle, isocétyle, stéaryle, isostéaryle, oléyle, octyldodécyle, béhényle, docosényle, décyltétradécyle et benzyle, hydroxyéthyle, hydroxypropyle, hydroxybutyle et 2,3-dihydroxypropyle.

3. Composition selon la revendication 1 ou 2, dans laquelle X est Cl ou Br.

4. Une composition selon la revendication 3, dans laquelle l'agent tensioactif est choisi parmi le chlorure de triméthylcétylammonium, le chlorure de triméthylstéarylammonium, le chlorure de triméthylbéhénylammonium, le chlorure de triméthyldocosénylammonium, le chlorure de diméthyldicétylammonium, le chlorure de diméthyldistéarylammonium, le chlorure de triméthyloctyldécylammonium et le chlorure de triméthyldécyltétradécylammonium.

5. Une composition selon l'une quelconque des revendications, précédentes dans laquelle l'oxyde d'amine est choisi parmi l'oxyde N,N-diméthylcétylamine, l'oxyde de N,N-diméthylstéarylamine, l'oxyde de N,N-di(2-hydroxyéthyl)stéarylamine, l'oxyde de N-méthyldistéarylamine, l'oxyde de N,N-diméthylbéhénylamine, l'oxyde de N,N-diméthyldocosénylamine, l'oxyde de N,N-diméthyloctyldodécylamine et l'oxyde de N,N-diméthyldécyltétradécylamine.

6. Une composition selon l'une quelconque des revendications précédentes dans laquelle l'aminoacide est choisi parmi la glycine, l'alanine, la valine, la leucine, l'isoleucine, la sérine, la thréonine, la phénylalanine, la tyrosine, le tryptophane, la cystéine, la cystine, la méthionine, la proline, l'hydroxyproline, l'acide asparaginique, l'acide glutamique, l'asparagine ou la glutamine.

7. Une composition selon l'une quelconque des revendications précédentes dans laquelle le sel est choisi parmi les aluns et les sels de sodium, potassium, magnésium et calcium des acides chlorhydrique, sulfurique et phosphorique.

8. Un procédé qui consiste à rincer les cheveux avec une composition liquide ayant un lustre nacré comprenant les composants (1), (2), (3) et (4) tels que définis à l'une quelconque des revendications précédentes.